# EUROPÄISCHE PATENTSCHRIFT

(11) **EP 1 420 751 B1**
(45) Veröffentlichungstag und Bekanntmachung des Hinweises auf die Patenterteilung: **04.10.2006**
(21) Anmeldenummer: 02747466.7
(22) Anmeldetag: 12.07.2002
(51) Int. Cl.: A61Q 5/10, A61K 8/86, A61K 8/87, C08L 75/04

(54) **FÄRBEMITTEL FÜR KERATINFASERN**
COLOURING AGENT FOR KERATIN FIBRES
COLORANTS POUR FIBRES DE KERATINE

(30) Priorität: 28.08.2001 DE 20114179 U
(43) Veröffentlichungstag der Anmeldung: 26.05.2004
(73) Patentinhaber: Wella Aktiengesellschaft, 64274 Darmstadt (DE)
(72) Erfinder: SCHMENGER, Jürgen, 64331 Weiterstadt (DE); ABELS, Wilhelm, Simi Valley, CA 93065 (US); SCHMITT, Manfred, 64646 Heppenheim (DE); HESS, Gabriele, 64390 Erzhausen (DE); FRANK, Anke, 64347 Griesheim (DE)
(86) Internationale Anmeldenummer: PCT/EP2002/007763
(87) Internationale Veröffentlichungsnummer: WO 2003/017956

(56) Entgegenhaltungen:
- EP-A- 1 093 806
- WO-A-96/40815
- DE-U- 20 018 140
- FR-A- 2 802 089
- FR-A- 2 802 090
- FR-A- 2 802 092
- US-A- 6 156 076

## Beschreibung

Gegenstand der Erfindung sind Mittel zum Färben von Keratinfasern, insbesondere menschlichen Haaren, mit einem Gehalt an direktziehenden und/oder oxidativen Farbstoffen und einer speziellen Verdickerkombination sowie ein Verfahren zum Färben von Haaren unter Verwendung dieser Mittel.

Färbende Präparate liegen üblicherweise in Form von wässrigen -vorzugsweise verdickten- Lösungen oder Emulsionen vor und enthalten neben Farbstoffen beispielsweise Fettalkohole und/oder andere Ölkomponenten, Emulgatoren und Tenside, sowie gegebenenfalls Alkohole. Oxidationsfärbemittel bestehen in der Regel aus zwei Komponenten, (i) der die Farbstoffe enthaltenden Farbstoffträgermasse und (ii) der Oxidationsmittelzubereitung, die kurz vor dem Gebrauch miteinander vermischt und dann auf das zu färbende Haar aufgetragen werden. Je nach Viskosität und Mischungsverhältnis der beiden Komponenten ergibt sich beim Vermischen eine höhere oder niedrigere Viskosität. Eine gute Haftung des Färbemittels wird hierbei insbeondere durch eine höhere Viskosität des Färbemittels erzielt. Zudem benötigt der Friseur oft für seine Arbeiten höhere Viskositäten, beispielsweise bei speziellen Strähnen- oder Folientechniken sowie um gezielte Arbeiten mit dem Färbepinsel oder dem Akzentuierpinsel verrichten zu können.

Es bestand daher ein grosses Bedürfnis nach einer kostengünstigen Verdickung der Farbstoffträgermasse, welche eine gute Vermischbarkeit der Farbstoffträgermasse mit dem Oxidationsmittel gewährleistet und Färbemittel mit guten Haftungseigenschaften und Färbeeigenschaften ergibt.

Die Verwendung von nichtionischen, amphiphilen Assoziativverdickern zur Verdickung von Haarfärbemitteln ist aus der WO 01/41723 bekannt. Die dort beschriebenen Mittel sind jedoch hinsichtlich ihrer Viskosität und ihrer Haftung auf dem Haar sowie der färberischen Eigenschaften nicht in jeder Hinsicht befriedigend. So verschlechtern sich beispielsweise die viskositätsbildenden Eigenschaften bereits nach einer Lagerzeit von einigen Wochen. Weiterhin weisen die in der WO 01/41723 beschriebenen Färbemittel einen honigartigen Charakter auf, der die Farbmasse vom Haar herablaufen läßt. Ebenfalls nachteilig sind die Unverträglichkeiten hoher Farbstoffkonzentrationen mit den Assoziativverdickern.

Hierzu wurde nunmehr gefunden, dass die vorgenannten Nachteile der Assoziatiwerdicker durch die Verwendung einer Kombination dieser Assoziativverdicker mit einem Fettalkohol und einem weiteren Verdickungsmittel auf Basis eines hydrophobierten, modifizierten nichtionischen Polyols behoben werden können.

Gegenstand der vorliegenden Erfindung ist daher ein Mittel zur Färbung von Keratinfasern, insbesondere Haaren, auf der Basis von Oxidationsfarbstoffvorstufen und/oder direktziehenden Farbstoffen, welches dadurch gekennzeichnet ist, dass es in einem geeigneten kosmetischen Träger eine Kombination aus
(a) mindestens einem nichtionischen, amphiphilen Assoziatiwerdicker,
(b) mindestens einem bei 25°C flüssigen Fettalkohol und
(c) ein PEG-150/Stearyl Alcohol/SMDI Copolymer enthält.

Der nichtionische, amphiphile Assoziatiwerdicker ist in der erfindungsgemäßen Zusammensetzung (jeweils bezogen auf die Aktivsubstanz) vorzugsweise in einer Menge von etwa 0,01 bis 2,5 Gewichtsprozent, insbesondere von etwa 0,1 bis 1 Gewichtsprozent enthalten.

Als nichtionischer, amphiphiler Assoziatiwerdicker wird ein Polymer eingesetzt, welches sowohl hydrophile als auch hydrophobe Gruppen enthält. Assoziativverdicker sind wasserlösliche Polymere und haben tensidartige hydrophobe Bestandteile, welche in der Lage sind, sich in einem hydrophilen, insbesondere wässrigen Medium sowohl mit sich selbst als auch mit anderen hydrophoben Stoffen zu assoziieren, das heisst in Wechselwirkung zu treten. Durch das daraus resultierende assoziative Netzwerk wird das Medium verdickt oder geliert. Typischerweise werden Assoziatiwerdicker durch Polymerisation von Polyethylenoxid-Prepolymeren und mindestens zweifach funktionellen, polykondensierbaren Stoffen wie zum Beispiel Isocyanaten hergestellt, wobei Monohydroxyverbindungen oder Dihydroxyverbindungen mit großen Aryl- Gruppen, Alkyl- Gruppen oder Aryl/Alkyl-Gruppen eingebaut werden, um die hydrophobe Modifikation bereitzustellen. Bevorzugte Assoziatiwerdicker sind daher hydrophob modifizierte Polyalkylenglykole. Hierbei wird der hydrophile Bestandteil durch Polyoxyalkyleneinheiten, vorzugwseise Polyoxyethyleneinheiten aber auch Polyoxypropyleneinheiten oder deren Gemisch gebildet. Der hydrophobe Bestandteil wird vorzugsweise aus Kohlenwasserstoffgruppen, beispielsweise langkettigen Alkylgruppen, Alkylarylgruppen oder Arylalkylgruppen gebildet.

Besonders bevorzugte Assoziatiwerdicker sind hydrophob modifizierte Aminoplast-Polyether-Copolymere. Bezüglich deren Struktur und Herstellung wird auf die WO 96/40815 verwiesen. In der WO 96/40815 werden wasserdispergierbare oder wasserlösliche Copolymere beschrieben, welche die Reaktionsprodukte sind einer säurekatalysierten Polykondensation von mindestens zweifach funktionellen Aminoplastmonomeren und mindestens zweifach funktionellen Alkylenpolyethern sowie einfach funktionellen Verbindungen mit hydrophoben Gruppen. Geeignete Aminoplaste sind der Figur 1 der WO 96/40815 zu entnehmen. Besonders bevorzugt sind hierbei die Glycolurilderivate der Formel X der WO 96/40815. Geeignete Alkylenpolyether sind der Figur 2 der WO 96/40815 zu entnehmen. Bevorzugte Alkylenpolyether sind Polyethylenoxiddiole. Diese können einen Ethoxylierungsgrad von 20 bis 500, vorzugsweise 50 bis 350, besonders bevorzugt von 100 bis 250 haben. Geeignete einfach funktionelle Verbindungen mit hydrophoben Gruppen sind diejenigen der Formel XIV der WO 96/40815.

Erfindungsgemäß geeignete Assoziatiwerdicker sind vorzugsweise ausgewählt aus Polymeren der allgemeinen Formel (I) 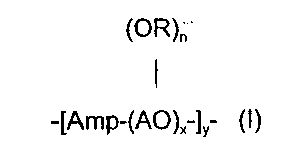 wobei Amp ein Aminoplastmonomer oder den Rest eines Aminoplastoligomers oder Aminoplastpolymers bedeutet, AO für eine Alkylenoxidgruppe steht, R für Wasserstoff, eine C1-C4-Alkylgruppe oder eine C1-C4-Acylgruppe steht und x und y Zahlen größer 1 sind.

Besonders bevorzugt sind die Reaktionsprodukte der säurekatalysierten Polykondensation von (a) Glykolurilen der allgemeinen Formel (II), 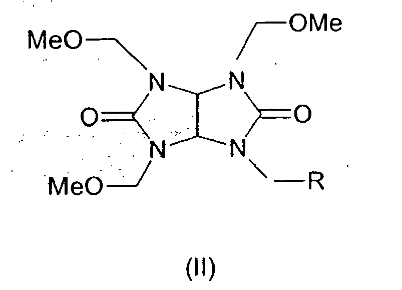 wobei R für H oder vorzugsweise für OMe steht, mit (b) Polyethylenoxiddiolen eines Ethoxylierungsgrades von 20 bis 500, vorzugsweise 50 bis 350, besonders bevorzugt von 100 bis 250 sowie (c) eines gegebenenfalls ethoxylierten hydrophoben Alkohols, Alkylphenols, Thiols, Carboxamids, Carbamats oder einer hydrophoben Carbonsäure, wie sie auf den Seiten 17 bis 19 der WO 96/40815 beschrieben sind. Ein besonders bevorzugtes Glykoluril ist das 1,3,4,6-Tetramethoxymethylglycoluril (TMMG).

Besonders geeignete Assoziativverdicker sind solche mit den INCI-Bezeichnungen Polyether-1, PEG-180/Octoxynol-40/TMMG Copolymer und PEG-180/Laureth-50/TMMG Copolymer und werden beispielsweise von der Firma Süd-Chemie AG, München/Deutschland unter den Handelsbezeichnungen Pure-Thix® HH, HL, L, M, TX-1442, TX-1450, TX-1451, TX-1452 und TX-1499 vertrieben.

Als Fettalkohol können prinzipiell alle bei Raumtemperatur (etwa 25 °C) flüssigen gesättigten oder ungesättigten, verzweigten oder geradkettigen Fettalkohole verwendet werden. Als geeignete Fettalkohole können insbesondere Decanol, Undecanol, Laurylalkohol, 2-Octyl-1-dodecanol, Oleylalkohol, Linoleylalkohol und Linolenylalkohol genannt werden, wobei Laurylalkohol, 2-Octyl-1-dodecanol und Oleylalkohol besonders bevorzugt sind.

Die Einsatzmenge des Fettalkohols in dem erfindungsgemäßen Färbemittel beträgt vorzugsweise etwa 0,1 bis 20 Gewichtsprozent, insbesondere etwa 0,5 bis 10 Gewichtsprozent.

Als Komponente (c) wird ein Copolymer aus PEG-150, einem gesättigten Methylen-diphenyldiisocyanat und Stearylalkohol (INCI-Name: PEG-150/Stearyl AlcohoI/SMDI Copolymer), welches beispielsweise unter dem Handelsnamen Aculyn 46 von der Firma ISP, Wayne/USA vertrieben wird, eingesetzt; wobei die Einsatzmenge dieses Verdickungsmittels vorzugsweise etwa 0,01 bis 2 Gewichtsprozent, und insbesondere etwa 0,05 bis 1 Gewichtsprozent, beträgt.

Das erfindungsgemäße Färbemittel enthält vorzugsweise Oxidationsfarbstoffvorstufen, bei denen die Färbung unter Einwirkung von Oxidationsmitteln, wie zum Beispiel Wasserstoffperoxid, oder in Gegenwart von Luftsauerstoff erzeugt wird.

Als geeignete Oxidationsfarbstoffvorstufen können beispielsweise die folgenden Entwicklersubstanzen und Kupplersubstanzen und mit sich selbst kuppelnden Verbindungen genannt werden:
(i) Entwicklersubstanzen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diamino-biphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylaminoanilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyeth-yl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1 H-pyrazol, 2-Amino-phenol, 2-Amino-6-methyl-phenol, 2-Amino-5-methyl-phenol, allein oder im Gemisch miteinander.
(ii) Kupplersubstanzen: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxy-ethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, allein oder im Gemisch miteinander.
(iii) Mit sich selbst kuppelnde Verbindungen: 2-Amino-5-methylphenol, 2-Amino-6-methylphenol, 2-Amino-5-ethoxyphenol oder 2-Propyl-amino-5-aminopyridin.

Die Gesamtmenge der in dem erfindungsgemäßen Mittel enthaltenen Oxidationsfarbstoffvorstufen beträgt etwa 0,01 bis 12 Gewichtsprozent, insbesondere etwa 0,2 bis 6 Gewichtsprozent.

Zur Erzielung bestimmter Farbnuancen können ferner auch übliche natürliche und/oder synthetische direktziehende Farbstoffe, beispielsweise sogenannte Pflanzenfarbstoffe wie Henna oder Indigo,Triphenylmethanfarbstoffe, aromatische Nitrofarbstoffe, Azofarbstofte, Chinonfarbstoffe, kationische oder anionische Farbstoffe, in dem Färbemittel enthalten sein.

Als geeignete synthetische Farbstoffe können beispielsweise genannt werden: 1,4-Bis[(2-hydroxyethyl)amino]-2-nitrobenzol, 1-(2-Hydroxyethyl)-amino-2-nitro-4-[di(2-hydroxyethyl)-amino]-benzol (HC Blue No. 2), 1-Amino-3-methyl-4-[(2-hydroxyethyl)-amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxy-ethyl)-amino]-2-nitrobenzol (HC Blue No. 10), 1-[(2,3-Dihydroxypropyl)-amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxy-propyl)amino]-2-nitrobenzol (HC Blue No. 6), 2-((4-Amino-2-nitrophenyl)-amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxy-ethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetra-hydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxy-ethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxy-ethyl)-amino]-1-methoxy-5-nitrobenzol, 2-Amino-3-nitrophenol, 1-(2-Hydroxyethoxy)-3-methylamino-4-nitrobenzol, 2,3-(Dihydrox,y-propoxy)-3-methyl-amino-4-nitrobenzol, 2-[(2-Hydroxyethyl)amino)-5-nitrophenol (HC Yellow No. 11), 3-[(2-Aminoethyl)-amino]-1-methoxy-4-nitrobenzol-hydrochlorid (HC Yellow No.9), 1-[(2-Ureido-ethyl)amino]-4-nitrobenzol, 4-[(2,3-Di-hydroxypropyl)amino]-3-nitro-1-trifluormethyl-benzol (HC Yellow No. 6), 1-Chlor-2,4-bis[(2-hydroxyethyl)-amino]-5-nitrobenzol (HC Yellow No. 10), 4-[(2-Hydroxy-ethyl)amino]-3-nitro-1-methylbenzol, 1-Chlor-4-[(2-hydroxy-ethyl)amino]-3-nitrobenzol (HC Yellow No. 12), 4-[(2-Hydroxyethyl)-amino]-3-nitro-1-trifluormethylbenzol (HC Yellow No. 13), 4-[(2-Hydroxy-ethyl)amino]-3-nitro-benzonitril (HC Yellow No. 14), 4-[(2-Hydroxyethyl)-amino]-3-nitro-benzamid (HC Yellow No. 15), 1,4-Di[(2,3-dihydroxy-propyl)ämino]-9,10-anthrachinon, 1-[(2-Hydroxy-ethyl)amino]-4-methyl-amino-9,10-anthrachinon (Cl61505, Disperse Blue No. 3), 2-[(2-Amino-ethyl)amino]-9,10-anthrachinon (HC Orange No. 5), 1-Hydroxy-4-[(4-methyl-2-sulfophenyl)amino]-9,10-anthrachinon, 1-[(3-Aminopropyl)-amino]-4-methylamino-9,10-anthrachinon (HC Blue No. 8),1-[(3-Amino-propyl)-amino]-9,10-anthrachinon (HC Red No. 8), 1,4-Diamino-2-methoxy-9,10-anthrachinon (Cl62015, Disperse Red No. 11, Solvent Violet No. 26), 1,4-Dihydroxy-5,8-bis[(2-hydroxyethyl)amino]-9,10-anthrachinon (Cl62500, Disperse Blue No. 7, Solvent Blue No. 69), 9-(Dimethylamino)-benzo[a]-phenoxazin-7-ium-chlorid (Cl51175; Basic Blue No. 6), Di[4-(diethyl-amino)phenyl][4-(ethylamino)naphthyl]-carbenium-chlorid (Cl42595; Basic Blue No. 7), 3,7-Di(dimethylamino)-phenothiazin-5-ium-chlorid (Cl52015; Basic Blue No. 9), Di[4-(dimethyl-amino)phenyl][4-(phenylamino)naphthyl]-carbenium-chlorid (Cl44045; Basic Blue No. 26), 2-[(4-(Ethyl(2-hydroxy-ethyl)amino)phenyl)azo]-6-methoxy-3-methyl-benzothiazolium-methylsulfat (Cl11154; Basic Blue No. 41), 8-Amino-2-brom-5-hydroxy-4-imino-6-[(3-(trimethylammonio)phenyl)-amino]-1(4H)-naphthalinon-chlorid (Cl56059; Basic Blue No. 99), Bis[4-(dimethyl-amino)phenyl][4-(methyl-amino)phenyl]carbenium-chlorid (Cl42535; Basic Violet No. 1), Tris[4-(dimethylamino)phenyl]carbenium-chlorid (Cl42555; Basic Violet No. 3), 2-[3,6-(Diethylamino)-dibenzopyranium-9-yl]-benzoesäure-chlorid (CI45170; Basic Violet No. 10), Di(4-aminophenyl)-(4-amino-3-methyl-phenyl)carbenium-chlorid (Cl42510; Basic Violet No. 14), 1,3-Bis[(2,4-diamino-5-methylphenyl)azo]-3-methylbenzol (Cl21010; Basic Brown No. 4), 1-[(4-Aminophenyl)azo]-7-(trimethylammonio)-2-naphthol-chlorid (CI12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethyl-ammonio)-2-naphthol-chlorid (CI12251; Basic Brown No. 17), 3,7-Diamino-2,8-dimethyl-5-phenyl-phenazinium-chlorid (Cl50240; Basic Red No. 2), 1,4-Dimethyl-5-[(4-(dimethylamino)phenyl)-azo]-1,2,4-triazolium-chlorid (Cl11055; Basic Red No. 22), 2-Hydroxy-1-[(2-methoxy-phenyl)azo]-7-(trimethylammonio)-naphthalin-chlorid (Cl12245; Basic Red No. 76), 2-[2-((2,4-Dimethoxyphenyl)amino)ethenyl]-1,3,3-trimethyl-3H-indol-1-ium-chlorid (Cl48055; Basic Yellow No. 11), 3-Methyl-1-phenyl-4-[(3-(trimethylammonio)-phenyl)azo]-pyrazol-5-on-chlorid (Cl12719; Basic Yellow No. 57), Bis[4-(diethylamino)phenyl]phenylcarbenium-hydrogensulfat(1:1) (CI42040; Basic Green No. 1), 1-[Di(2-hydroxyethyl)-amino]-3-methyl-4-[(4-nitro-phenyl)azo]-benzol (CI11210, Disperse Red No. 17), 4-[(4-Aminophenyl)-azo]-1-[di(2-hydroxyethyl)amino]-3-methyl-benzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (Cl15985; Food Yellow No. 3; FD&C Yellow No. 6), 2,4-Dinitro-1-naphthol-7-sulfonsäure-dinatriumsalz (Cl10316; Acid Yellow No. 1; Food Yellow No. 1), 2-(Indan-1,3-dion-2-yl)chinolin-x,x-sulfonsäure (Gemisch aus Mono- und Disulfonsäure) (Cl47005;D&C Yellow No. 10; Food Yellow No. 13; Acid Yellow No. 3), 5-Hydroxy-1-(4-sulfophenyl)-4-[(4-sulfophenyl)-azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 5-[(2,4-Dinitrophenyl)-amino]-2-phenylamino-benzolsulfonsäure-natriumsalz (Cl10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (Cl15510; Acid Orange No. 7), 4-[(2,4-Dihydroxy-3-[(2,4-dimethylphenyl)azo]phenyl)azo]-benzolsulfonsäure-natriumsalz (Cl20170; Acid Orange No. 24), 4-Hydroxy-3-[(4-sulfonaphth-1-yl)azo]-1-naphthalin-sulfonsäure-dinatriumsalz (Cl14720; Acid Red No. 14), 6-Hydroxy-5-[(4-sulfonaphth-1-yl)azo]-2, 4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (Cl45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (Cl45100; Acid Red No. 52), 8-[(4-(Phenylazo)-phenyl)azo]-7-naphthol-1,3-disulfonsäure-dinatriumsalz (C127290; Acid Red No. 73), 2',4',5',7'-Tetrabrom-3',6'-dihydroxyspiro-[isobenzofuran-1(3H),9'-[9H]xanthen]-3-on-dinatriumsalz (Cl45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (C145410; Acid Red No. 92), 3',6'-Dihydroxy-4',5'-diiodospiro[isobenzofuran-1 (3H),9'(9H)-xanthen]-3-on-dinatriumsalz (Cl45425; Acid Red No. 95), (2-Sulfophenyl)di[4-(ethyl((4-sulfophenyl)methyl)amino)phenyl]-carbenium-dinatriumsalz, betain (Cl42090; Acid Blue No. 9; FD&C Blue No. 1), 1,4-Bis[(2-sulfo-4-methylphenyl)amino]-9,10-anthrachinon-dinatriumsalz (Cl 61570; Acid Green No. 25), Bis[4-(dimethylamino)phenyl]-(3,7-disulfo-2-hydroxy-naphth-1-yl)carbenium-inneres Salz, mononatriumsalz (Cl44090; Food Green No. 4; Acid Green No. 50), Bis[4-(diethylamino)phenyl](2,4-disulfophenyl)carbenium-inneres salz, Natriumsalz (2:1) (Cl42045; Food Blue No. 3; Acid Blue No. 1), Bis[4-(diethylamino)phenyl](5-hydroxy-2,4-disulfophenyl)carbenium-inneres salz, Calciumsalz (2:1) (Cl42051; Acid Blue No. 3), 1-Amino-4-(cyclohexylamino)-9,10-anthrachinon-2-sulfonsäure-natriumsalz (CI62045; Acid Blue No. 62),2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (Cl60730; D&C Violett No. 2; Acid Violet No. 43), Bis[3-nitro-4-[(4-phenylamino)-3-sulfo-phenylamino]-phenyl]-sulfon (Cl10410; Acid Brown No. 13), 5-Amino-4-hydroxy-6-[(4-nitrophenyl)azo]-3-(phenylazo)-2,7-naphthalindisulfonsäure-dinatriumsalz (Cl20470; Acid Black No. 1), 3-Hydroxy-4-[(2-hydroxynaphth-1-yl)azo)-7-nitro-1-naphthalin-sulfonsäure-chromkomplex (3:2) (Cl15711; Acid Black No. 52), 3-[(2,4-Dimethyl-5-sulfophenyl)azo]-4-hydroxy-1-naphthalin-sulfonsäure-dinatriumsalz (Cl14700; Food Red No. 1; FD&C Red No. 4), 4-(Acetylamino)-5-hydroxy-6-[(7-suffo-4-[(4-sulfophenyl)azo]naphth-1-yl)azo]-1,7-naphthalindisulfonsäure-tetranatriumsalz (Cl28440; Food Black No. 1) und 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red No. 195), alleine oder in Kombination miteinander.

Die Gesamtmenge der direktziehenden Farbstoffe beträgt in dem erfindungsgemässen Mittel etwa 0,01 bis 7 Gewichtsprozent, vorzugsweise etwa 0,2 bis 4 Gewichtsprozent.

Weitere zur Haarfärbung bekannte und übliche Farbstoffe, die in dem erfindungsgemässen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben.

Obwohl Oxidationsfärbemittel bevorzugt sind, ist es selbstverständlich ebenfalls möglich, dass das erfindungsgemässe Färbemittel in Form eines nicht-oxidativen Färbemittels auf Basis der vorstehend genannten direktziehenden Farbstoffen vorliegt.

Darüberhinaus können in dem erfindungsgemäßen Mittel Antioxidantien wie zum Beispiel Ascorbinsäure, Thioglykolsäure oder Natriumsulfit, sowie Komplexbildner für Schwermetalle, beispielsweise Ethylendiamino-tetraacetat oder Nitriloessigsäure, in einer Menge von bis zu etwa 0,5 Gewichtsprozent enthalten sein. Parfümöle können in der erfindungsgemäßen Farbträgermasse in einer Menge von bis zu etwa 1 Gewichtsprozent enthalten sein. Selbstverständlich kann das vorstehend beschriebene Haarfärbemittel gegebenenfalls weitere, für Haarfärbemittel übliche Zusätze, wie zum Beispiel Konservierungsstoffe; Antioxidantien, beispielsweise Natriumsulfit, Thioglykolsäure oder Ascorbinsäure; Komplexbildner; Lösungsmittel wie Wasser, niedere aliphatische Alkohole, beispielsweise aliphatische Alkohole mit 1 bis 4 Kohlenstoffatomen wie Ethanol, Propanol und Isopropanol, oder Glykole wie Glycerin und 1,2-Propylenglykol; Netzmittel oder Emulgatoren aus den Klassen der anionischen, kationischen, amphoteren oder nichtionogenen oberflächenaktiven Substanzen; weiterhin Weichmacher; Vaseline; Silikonöle, Paraffinöl und Fettsäuren sowie außerdem Pflegestoffe, wie kationische Harze, Lanolinderivate, Cholesterin, Vitamine, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,1 bis 30 Gewichtsprozent und die Pflegestoffe in einer Konzentration von 0,1 bis 5,0 Gewichtsprozent. Besonders vorteilhaft ist hierbei der Zusatz von nicht-ionischen und/oder anionischen Tensiden oder Emulgatoren, wie zum Beispiel Fettalkoholsulfaten, insbesondere Laurylsulfat und Natriumcocoylsulfat, oxethylierten Fettalkoholsulfaten, insbesondere Natriumlaurylethersulfaten mit 2 bis 4 Ethylenoxideinheiten im Molekül, oxethylierten Fettsäureestern, oxethylierten Nonylphenolen, oxethylierten Fettalkoholen, Alkylbenzolsulfonaten oder Fettsäurealkanolamiden, in einer Gesamtmenge von etwa 0,1 bis 30 Gewichtsprozent, vorzugsweise 0,2 bis 15 Gewichtsprozent.

Der pH-Wert des erfindungsgemäßen Färbemittels liegt bei nicht-oxidativen Färbemitteln auf der Basis von direktziehenden Farbstoffen im Bereich von etwa 5 bis 10, vorzugsweise 6 bis 9, während bei oxidativen Färbemitteln auf der Basis von Oxidationsfarbstoffvorstufen der pH-Wert in einem Bereich von etwa 6 bis 12, vorzugsweise 9 bis 11, liegt, wobei der pH-Wert des gebrauchsfertigen Oxidationshaarfärbemittels (das heißt der Mischung des erfindungsgemäßen Haarfärbemittels mit dem Oxidationsmittel) etwa 5,5 bis 10, vorzugsweise 6 bis 9, beträgt.

Je nach Zusammensetzung und gewünschtem pH-Wert des Färbemittels erfolgt die Einstellung des pH-Wertes vorzugsweise mit Ammoniak oder organischen Aminen, wie zum Beispiel Glucaminen, Aminomethylpropanol, Monoethanolamin oder Triethanolamin, anorganischen Basen, beispielsweise Natriumhydroxid, Kaliumhydroxid, Natriumcarbonat oder Calciumhydroxid, beziehungsweise organischen oder anorganischen Säuren, wie zum Beispiel Milchsäure, Zitronensäure, Essigsäure oder Phosphorsäure.

Das erfindungsgemäße Mittel wird vorzugsweise in Form einer wässrigen oder wässrig-alkoholischen Zubereitung, beispielsweise als verdickte Lösung, als Emulsion, als Creme oder als Gel, konfektioniert. Es ist jedoch prinzipiell auch möglich, das Färbemittel erst unmittelbar vor der Anwendung durch Zusatz der erfindungsgemäßen 3er-Kombination zu verdicken.

Für die Anwendung zur oxidativen Färbung vermischt man das vorstehend beschriebene Färbemittel unmittelbar vor dem Gebrauch mit einem Oxidationsmittel und trägt eine für die Färbung ausreichende Menge, in der Regel etwa 60 bis 200 Gramm, der gebrauchsfertigen Zubereitung auf die Faser auf.

Sofern das erfindungsgemäße Färbemittel keine Oxidationsfarbstoffvorstufen enthält beziehungsweise Oxidationsfarbstoffvorstufen enthält, welche mit Luftsauerstoff leicht oxidierbar sind, kann es ohne vorheriges Vermischen mit einem Oxidationsmittel direkt auf die Keratinfaser aufgetragen werden.

Als Oxidationsmittel zur Entwicklung der Färbung kommen hauptsächlich Wasserstoffperoxid oder dessen Additionsverbindungen an Harnstoff, Melamin oder Natriumborat in Form einer 1- bis 12prozentigen, vorzugsweise 1,5- bis 6prozentigen wässrigen Lösung in Betracht. Das Mischungsverhältnis von Färbemittel zu Oxidationsmittel ist abhängig von der Konzentration des Oxidationsmittels und beträgt in der Regel etwa 5:1 bis 1:2, vorzugsweise 1:1, wobei der Gehalt an Oxidationsmittel in der gebrauchsfertigen Zubereitung vorzugsweise etwa 0,5 bis 8 Gewichtsprozent, insbesondere 1 bis 4 Gewichtsprozent, beträgt.

Man läßt das gebrauchsfertige Färbemittel bei 15 bis 50 °C etwa 10 bis 45 Minuten, vorzugsweise etwa 15 bis 30 Minuten lang, auf die Keratinfaser (zum Beispiel menschliche Haare) einwirken, spült sodann die Faser mit Wasser aus und trocknet sie. Gegebenenfalls wird im Anschluß an diese Spülung mit einem Shampoo gewaschen und eventuell mit einer schwachen organischen Säure, wie zum Beispiel Weinsäure, nachgespült. Anschließend wird die Keratinfaser getrocknet.

Falls erforderlich kann die Viskosität des erfindungsgemässen Oxidationsfärbemittels auch noch nach dem Vermischen mit dem Oxidationsmittel durch Zusatz der erfindungsgemäßen Kombination der Komponenten (a) bis (c) nachträglich ohne weiteres erhöht werden kann, wodurch einfachere und kostengünstigere Basisformulierungen möglich sind. Ebenfalls ist es möglich, das Färbemittel erst unmittelbar vor dem Gebrauch (vor, nach oder während dem Vermischen mit dem Oxidationsmittel) durch Zusatz der erfindungsgemäßen Kombination der Komponenten (a) bis (c) zu verdicken.

Ein mit der erfindungsgemäßen Zusammensetzung hergestelltes Färbemittel erfüllt die in Bezug auf die Hafteigenschaften, das Auftrageverhalten und die Viskositätseinstellung gestellten Anforderungen in hervorragenderer Weise und ist spürbar leichter aufzutragen. Weiterhin besitzen die erfindungsgemäßen Färbemittel eine gleichmäßige Konsistenz und eine kosmetische Anmutung. Insbesondere ist die sehr gute Viskosität und die hervorragende Stabilität des erfindungsgemäßen Mittels sowie dessen ausgezeichnete Haftung auf dem Haar hervorzuheben. Weiterhin ermöglicht die Verwendung der erfindungsgemäßen Kombination aus (a) mindestens einem nichtionischen, amphiphilen Assoziatiwerdicker, (b) mindestens einem bei 25°C flüssigen Fettalkohol und (c) ein PEG-150/Stearyl Alcohol/SMDI Copolymer eine Variation des Gewichtsverhältnisses von Färbemittel und Oxidationsmittel über einen breiten Bereich (zum Beispiel 1:1 bis 1:2:5) ohne nennenswerte Beeinträchtigung der Viskosität und Haftungseigenschaften des gebrauchsfertigen Oxidationsfärbemittels.

Die nachfolgenden Beispiele sollen den Gegenstand der Erfindung näher erläutern ohne diesen hierauf zu beschränken.

### Beispiele

### Beispiel 1: Oxidationshaarfärbemittel, flüssig

| | |
|---|---|
| 0,2000 g | PEG-180/Laureth-50/TMMG Copolymer (entspricht 1 g Pure Thix® TX-1450 (20 %ig) der Firma Süd-Chemie/USA) |
| 0,2850 g | PEG-150/Stearyl Alcohol/SMDI Copolymer (entspricht 1,5 g Aculyn 46 (19 %ig) der Firma ISP/USA) |
| 5,0000 g | 2-Octyl-1-dodecanol (Eutanol G der Firma Cognis/ Deutschland) |
| 15,0000 g | Ölsäure |
| 10,0000 g | Natriumlaurylalkohol-diglykolethersulfat (28 %ige wässrige Lösung) |
| 1,3620 g | 4-Aminophenol |
| 0,5000 g | 1=Naphthol |
| 0,0136 g | Resorcin |
| 0,0034 g | 2-Amino-6-chlor-4-nitrophenol |
| 12,0000 g | Ammoniak, 25 %ige wässrige Lösung |
| 1,0000 g | Ethylendiaminoteraacetat-Dinatriumsalz |
| 1,0000 g | Ascorbinsäure |
| 15,0000 g | Isopropanol |
| ad 100,0000 g | Wasser |

50 g des vorstehenden Haarfärbemittels werden unmittelbar vor Gebrauch mit 50 g einer 6prozentigen wässrigen Wasserstoffperoxidlösung vermischt. Es wird eine homogene, kosmetisch anmutende, optimal verdickte Färbezubereitung erhalten. Das so erhaltene Gemisch wird anschließend auf blonde Naturhaare aufgetragen. Nach einer Einwirkungszeit von 30 Minuten bei 40 °C wird das Haar mit Wasser gespült und getrocknet. Das Haar erhält eine leuchtende, kupferrote Färbung.

### Beispiel 2: Gelförmiges Oxidationshaarfärbemittel zur Hellerfärbung

### Komponente (A): Flüssige Farbträgermasse

| | |
|---|---|
| 0,50 g | PEG-180/Octoxynol-40/TMMG Copolymer (entspricht 2,5 g Pure Thix® L (20 %ig) der Firma Süd-Chemie/USA) |
| 0,60 g | PEG-150/Stearyl Alcohol/SMDI Copolymer (entspricht 3,15 g Aculyn 46 (19 %ig) der Firma ISP/USA) |
| 10,00 g | Laurylalkohol |
| 6,00 g | Nonylphenol oxethyliert mit 4 Mol Ethylenoxid |
| 6,00 g | Ölsäure |
| 0,50 g | para-Phenylendiamin |
| 0,07 g | Resorcin |
| 5,00 g | Natriumlaurylalkohol-diglykolethersulfat, 28%ige wässrige Lösung |
| 1,00 g | Ethylendiamintetraessigsäure-Dinatriumsalz |
| 18,00 g | Ammoniak, 25 %ige wässrige Lösung |
| 8,00 g | Ethanol |
| ad 100 g | Wasser |

### Komponente (B): Wasserstoffperoxid-Emulsion

| | |
|---|---|
| 10,0 g | Cetylstearylalkohol |
| 1,5 g | Cholesterin |
| 4,0 g | Natriumlaurylalkohol-diglykolethersulfat, 28 %ige wässrige Lösung |
| 35,0 g | Wasserstoffperoxid, 35 %ige wässrige Lösung |
| 0,3 g | Parfüm |
| ad 100,0 g | Wasser |

Man vermischt vor dem Gebrauch 40 g der flüssigen Farbträgermasse (A) mit 80 g der Wasserstoffperoxid-Emulsion (B), entsprechend einem Mischungsverhältnis von (A):(B) von 1:2, und trägt 120 g dieses Gemisches auf graues, menschliches Haar auf. Nach einer Einwirkungszeit von 20 Minuten bei Raumtemperatur wird das Haar mit Wasser ausgespült getrocknet. Das so behandelte Haar ist vom Haaransatz bis zu den Haarspitzen gleichmäßig hellbraun gefärbt. Das erfindungsgemäße Mittel ist leicht auftragbar und läuft nicht vom Haar ab.

### Beispiel 3: Oxidationshaarfärbemittel, cremeförmig

| | |
|---|---|
| 0,10 g | PEG-180/Laureth-50/TMMG Copolymer (Pure Thix® TX-1450 der Firma Süd-Chemie/USA) |
| 0,30 g | PEG-150/Stearyl Alcohol/SMDI Copolymer (entspricht 1,6 g Aculyn 46 (19 %ig) der Firma ISP/USA) |
| 3,00 g | Oleylalkohol |
| 15,00 g | Cetylalkohol |
| 3,50 g | Natriumlaurylalkohol-diglykolethersulfat (28 %ige wässrige Lösung) |
| 3,00 g | Monoethanolamin |
| 1,30 g | 1-Methyl-2,5-diaminobenzol |
| 1,00 g | Bienenwachs |
| 0,65 g | Resorcin |
| 0,50 g | Keratinhydrolysat |
| 0,50 g | Seidenproteinhydrolysat |
| 0,50 g | 2-Amino-6-chlor-4-nitrophenol |
| 0,30 g | Ascorbinsäure |
| ad 100,00 g | Wasser |

50 g des vorstehenden Haarfärbemittels werden unmittelbar vor Gebrauch mit 50 g einer 12prozentigen wässrigen Wasserstoffperoxidlösung vermischt. Das erhaltene Gemisch wird anschließend auf blonde Naturhaare aufgetragen. Nach einer Einwirkungszeit von 30 Minuten bei 40 °C wird das Haar mit Wasser gespült und getrocknet. Es wird ein gleichmäßiger, kräftiger Braunton erhalten.

### Beispiel 4: (nicht-oxidative) Haartönung

| | |
|---|---|
| 1,8 g | PEG-180/Laureth-50/TMMG Copolymer (Pure Thix® TX-1450 der Firma Süd-Chemie/BRD) |
| 0,95 g | PEG-150/Stearyl Alcohol/SMDI Copolymer (entspricht 5,0 g Aculyn 46 (19 %ig) der Firma ISP/USA) |
| 6,0 g | Laurylalkohol |
| 5,0 g | Natriumlaurylsulfat |
| 1,5 g | 2-Amino-6-chlor-4-nitrophenol |
| 1,0 g | Monoethanolamin |
| 1,0 g | Bienenwachs |
| 0,5 g | Keratinhydrolysat |
| 0,3 g | Seidenproteinhydrolysat |
| 0,2 g | Glycin |
| ad 100,0 g | Wasser |

Es wird eine leicht gelige Färbemasse erhalten, die aufgrund ihrer hervorragenden Viskositätseigenschaften leicht und gleichmäßig aufgettragen werden kann sowie sehr gut auf dem Haar haftet. Nach einer Einwirkungszeit von 20 Minuten bei 20 °C wird das Haar mit lauwarmem Wasser ausgespült, zur Frisur gelegt und getrocknet. Das so behandelte Haar weist eine gleichmäßige, kräftig leuchtende goldorange Färbung auf.

### Beispiel 5: Oxidationshaarfärbemittel, cremeförmig

### Komponente (A): Farbträgermasse

| | |
|---|---|
| 2,0 g | PEG-180/Laureth-50/TMMG Copolymer (Pure Thix® TX-1450 der Firma Süd-Chemie/USA) |
| 2,0 g | PEG-150/Stearyl Alcohol/SMDI Copolymer (entspricht 10,5 g Aculyn 46 (19 %ig) der Firma ISP/USA) |
| 8,0 g | 2-Octyl-1-dodecanol (Eutanol G der Firma Cognis/ Deutschland) |
| 3,0 g | Natriumlaurylalkohol-diglykolethersulfat, 28 %ige wässrige Lösung |
| 2,8 g | 2,5-Diaminotoluolsulfat |
| 1,0 g | Resorcin |
| 0,4 g | m-Aminophenol |
| 0,2 g | 2-Amino-4-(2'-hydroxyethylamino)-anisolsulfat |
| 0,3 g | Ascorbinsäure |
| 0,1 g | Ethylendiamintetraessigsäure |
| 12,2 g | Ammoniak, 25 %ige wässrige Lösung |
| 2,0 g | Ethanol |
| ad 100,0 g | Wasser |

### Komponente (B): Wasserstoffperoxid - Emulsion

| | |
|---|---|
| 10,0 g | Cetylstearylalkohol |
| 1,5 g | Cholesterin |
| 4,0 g | Natriumlauryalkohol-diglykolethersulfat, 28%ige wässrige Lösung |
| 17,0 g | Wasserstoffperoxid, 35 %ige wässrige Lösung |
| 0,3 g | Parfüm |
| ad 100,0 g | Wasser |

Man vermischt vor dem Gebrauch 40 g der flüssigen Farbträgermasse (A) mit 80 g der Wasserstoffperoxid-Emulsion (B), entsprechend einem Mischungsverhältnis von (A):(B) von 1:2, und trägt 120 g dieses Gemisches auf graues, menschliches Haar auf. Nach einer Einwirkungszeit von 20 Minuten bei Raumtemperatur wird das Haar mit Wasser ausgespült getrocknet. Das so behandelte Haar hat einen gleichmäßigen, dunkelbraunen Ton angenommen. Das erfindungsgemäße Mittel haftet sehr gut auf dem Haar ohne abzulaufen.

### Beispiel 6: Vergleichsversuche

Ein Mittel gemäß Beispiel 1 wird mit den folgenden 3 nicht-erfindungsgemäßen Färbemitteln verglichen.
Mittel 6.1: Mittel gemäß Beispiel 1 in dem das PEG-150/Stearyl Alcohol/SMDI- Copolymer mengengleich durch das PEG-180/Laureth-50/TMMG-Copolymer ersetzt wurde.
Mittel 6.2: Mittel gemäß Beispiel 1 in dem das PEG-180/Laureth-50/TMMG-Copolymer mengengleich durch das PEG-150/Stearyl Alcohol/SMDI- Copolymer ersetzt wurde.
Mittel 6.3: Mittel gemäß Beispiel 1 in dem der Fettalkohol (2-Octyl-1-dodecanol) mengengleich durch eine andere Fettkomonente (Ölsäure) ersetzt wurde.

Während das erfindungsgemäße Mittel gemäß Beispiel 1 alle Erfordernisse hervorragend erfüllt (das heißt, nach dem Vermischen mit dem Oxidationsmittel sowohl eine ausgezeichnete Viskosität und eine hervorragende Haftung auf dem Haar besitzt als auch eine gleichmäßige Konsistenz, eine kosmetische Anmutung und hervorragende Auftrageeigenschaften aufweist), sind die nicht-erfindungsgemäßen Mittel in einem oder mehreren Punkten nicht befriedigend.

So besitzt das nicht-erfindungsgemäße Mittel 6.1 zwar eine gute Viskosität, jedoch läuft es aufgrund seiner schlechten Haftungseigenschaften vom Haar ab.
Das nicht-erfindungsgemäße Mittel 6.2 besitzt ebenfalls eine ausreichende Viskosität, jedoch ist die kosmetische Anmutung aufgrund der glibbrigen Konsistenz nicht befriedigend.
Das Mittel 6.3 ist nach dem Vermischen mit dem Oxidationsmittel wässrig dünn und besitzt keine ausreichende Viskosität.

Alle in der vorliegenden Anmeldung genannten Prozentangeaben stellen, soweit nicht anders angegeben, Gewichtsprozente dar.

## Patentansprüche

1. Mittel zur Färbung von Keratinfasern auf der Basis von Oxidationsfarbstoffvorstufen und/oder direktziehenden Farbstoffen, **dadurch gekennzeichnet, dass** es in einem geeigneten kosmetischen Träger eine Kombination aus (a) mindestens einem nichtionischen, amphiphilen Assoziatiwerdicker, (b) mindestens einem bei 25°C flüssigen Fettalkohol und (c) ein PEG-150/Stearyl Alcohol/SMDI Copolymer enthält, wobei die Komponenten (a) und (c) nicht identisch sind.

2. Mittel nach Anspruch 1, **dadurch gekennzeichnet, dass** der nichtionische, amphiphile Assoziatiwerdicker ausgewählt ist aus hydrophob modifizierten Polyalkylenglykolen.

3. Mittel nach Anspruch 1, **dadurch gekennzeichnet, dass** der nichtionische, amphiphile Assoziatiwerdicker ausgewählt ist aus hydrophob modifizierten Aminoplast-Polyether-Copolymeren.

4. Mittel nach Anspruch 1, **dadurch gekennzeichnet, dass** der nichtionische, amphiphile Assoziativverdicker ausgewählt ist aus Polymeren der allgemeinen Formel (I) 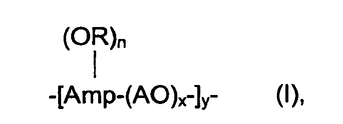 wobei Amp ein Aminoplastmonomer oder den Rest eines Aminoplastoligomers oder Aminoplastpolymers bedeutet, AO für eine Alkylenoxidgruppe steht, R für Wasserstoff, eine C1-C4-Alkylgruppe oder eine C1-C4-Acylgruppe steht und x und y Zahlen größer 1 sind.

5. Mittel nach Anspruch 1, **dadurch gekennzeichnet, dass** der nichtionische, amphiphile Assoziatiwerdicker ausgewählt ist aus Reaktionsprodukten der säurekatalysierten Polykondensation von (a) Glykolurilen der allgemeinen Formel (II), 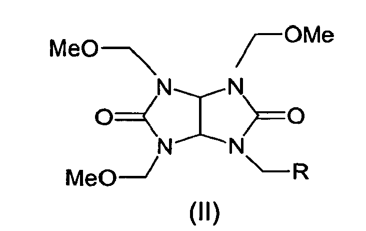 wobei R für H oder vorzugsweise für OMe steht, mit (b) Polyethylenoxiddiolen eines Ethoxylierungsgrades von 20 bis 500, sowie (c) eines gegebenenfalls ethoxylierten hydrophoben Alkohols, Alkylphenols, Thiols, Carboxamids, Carbamats oder einer hydrophoben Carbonsäure.

6. Mittel nach Anspruch 5, **dadurch gekennzeichnet, dass** das Glykoluril der Formel (II) das 1,3,4,6-Tetramethoxy-methylglycoluril ist.

7. Mittel nach einem der Ansprüche 1 bis 6, **dadurch gekennzeichnet, dass** der nichtionische, amphiphile Assoziativverdicker ausgewählt ist aus Polyether-1, PEG-180/Octoxynol-40/TMMG Copolymer und PEG-180/Laureth-50/TMMG Copolymer.

8. Mittel nach einem der Ansprüche 1 bis 7, **dadurch gekennzeichnet, dass** es den nichtionische, amphiphile Assoziatiwerdicker in einer Menge von 0,01 bis 2,5 Gewichtsprozent enthält.

9. Mittel nach einem der Ansprüche 1 bis 8, **dadurch gekennzeichnet, dass** der Fettalkohol ausgewählt ist aus Decanol, Undecanol, Laurylalkohol, 2-Octyl-1-dodecanol, Oleylalkohol, Linoleylalkohol und Linolenylalkohol.

10. Mittel nach einem der Ansprüche 1 bis 9, **dadurch gekennzeichnet, dass** es den Fettalkohol in einer Menge von 0,5 bis 20 Gewichtsprozent enthält.

11. Mittel nach einem der Ansprüche 1 bis 10, **dadurch gekennzeichnet, dass** es das PEG-150/Stearyl Alcohol/SMDI Copolymer in einer Menge von 0,01 bis 2 Gewichtsprozent enthält.

12. Mittel nach einem der Ansprüche 1 bis 11, **dadurch gekennzeichnet, dass** es 0,01 bis 12 Gewichtsprozent Oxidationsfarbstoffvorstufen enthält und vor der Anwendung mit einem Oxidationsmittel vermischt wird.

13. Mittel nach einem der Ansprüche 1 bis 11, **dadurch gekennzeichnet, dass** es 0,01 bis 12 Gewichtsprozent Oxidationsfarbstoffvorstufen enthält, welche durch Luftsauerstoff oxidierbar sind.

14. Mittel nach Anspruch 1, **dadurch gekennzeichnet, dass** es (a) 0,01 bis 2,5 Gewichtsprozent mindestens eines nichtionischen, amphiphilen Assoziatiwerdickers, (b) 0,1 bis 20 Gewichtsprozent mindestens eines bei 25°C flüssigen Fettalkohols und (c) 0,01 bis 2 Gewichtsprozent PEG-150/Stearyl Alcohol/SMDI Copolymer, wobei die Komponenten (a) und (c) nicht identisch sind, sowie mindestens eine Oxidationsfarbstoffvorstufe und ein Oxidationsmittel enthält, und gebrauchsfertig ist.

15. Mittel nach Anspruch 14, **dadurch gekennzeichnet, dass** es ein Haarfärbemittel ist.

## Claims

1. Agent for colouring keratin fibres based on oxidation dye precursors and/or direct dyes, **characterized in that** it comprises, in a suitable cosmetic carrier, a combination of (a) at least one nonionic, amphiphilic associative thickener, (b) at least one fatty alcohol liquid at 25°C and (c) a PEG-150/stearyl alcohol/SMDI copolymer, where components (a) and (c) are not identical.

2. Agent according to Claim 1, **characterized in that** the nonionic, amphiphilic associative thickener is chosen from hydrophobically modified polyalkylene glycols.

3. Agent according to Claim 1, **characterized in that** the nonionic, amphiphilic associative thickener is chosen from hydrophobically modified aminoplast-polyether copolymers.

4. Agent according to Claim 1, **characterized in that** the nonionic, amphiphilic associative thickener is chosen from polymers of the general formula (I) 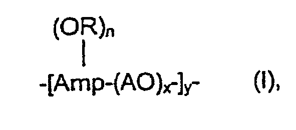 where Amp is an aminoplast monomer or the radical of an aminoplast oligomer or aminoplast polymer, AO is an alkylene oxide group, R is hydrogen, a C₁-C₄-alkyl group or a C1-C4-acyl group, and x and y are numbers greater than 1.

5. Agent according to Claim 1, **characterized in that** the nonionic, amphiphilic associative thickener is chosen from reaction products of the acid-catalysed polycondensation of (a) glycolurils of the general formula (II), 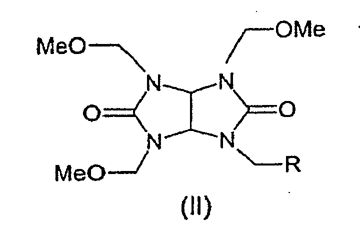 where R is H or preferably OMe, with (b) polyethylene oxide diols of a degree of ethoxylation of from 20 to 500, and (c) an optionally ethoxylated hydrophobic alcohol, alkylphenol, thiol, carboxamide, carbamate or a hydrophobic carboxylic acid.

6. Agent according to Claim 5, **characterized in that** the glycoluril of the formula (II) is 1,3,4,6-tetramethoxymethylglycoluril.

7. Agent according to one of Claims 1 to 6, **characterized in that** the nonionic, amphiphilic associative thickener is chosen from polyether-1, PEG-180/octoxynol-40/TMMG copolymer and PEG-180/laureth-50/TMMG copolymer.

8. Agent according to one of Claims 1 to 7, **characterized in that** it comprises the nonionic, amphiphilic associative thickener in an amount of from 0.01 to 2.5% by weight.

9. Agent according to one of Claims 1 to 8, **characterized in that** the fatty alcohol is chosen from decanol, undecanol, lauryl alcohol, 2-octyl-1-dodecanol, oleyl alcohol, linoleyl alcohol and linolenyl alcohol.

10. Agent according to one of Claims 1 to 9, **characterized in that** it comprises the fatty alcohol in an amount of from 0.5 to 20% by weight.

11. Agent according to one of Claims 1 to 10, **characterized in that** it comprises the PEG-150/stearyl alcohol/SMDI copolymer in an amount of from 0.01 to 2% by weight.

12. Agent according to one of Claims 1 to 11, **characterized in that** it comprises 0.01 to 12% by weight of oxidation dye precursors, and is mixed with an oxidizing agent prior to use.

13. Agent according to one of Claims 1 to 11, **characterized in that** it comprises 0.01 to 12% by weight of oxidation dye precursors which can be oxidized by atmospheric oxygen.

14. Agent according to Claim 1, **characterized in that** it comprises (a) 0.01 to 2.5% by weight of at least one nonionic, amphiphilic associative thickener, (b) 0.1 to 20% by weight of at least one fatty alcohol which is liquid at 25°C and (c) 0.01 to 2% by weight of PEG-150/stearyl alcohol/SMDI copolymer, where components (a) and (c) are not identical, and at least one oxidation dye precursor and an oxidizing agent, and is ready-to-use.

15. Agent according to Claim 14, **characterized in that** it is a hair colouring agent.

## Revendications

1. Agent pour la teinture de fibres kératiniques à base de précurseurs de colorants par oxydation et/ou de colorants montant directement sur la fibre, **caractérisé en ce qu'**il contient, dans un support cosmétique approprié, une combinaison (a) d'au moins un épaississant d'association non ionique, amphiphile, (b) d'au moins un alcool gras liquide à 25°C et (c) d'un copolymère de PEG-150/alcool stéarylique/SMDI, les composants (a) et (c) n'étant pas identiques.

2. Agent selon la revendication 1, **caractérisé en ce que** l'épaississant d'association non ionique, amphiphile est choisi parmi les polyalkylèneglycols modifiés de manière hydrophobe.

3. Agent selon la revendication 1, **caractérisé en ce que** l'épaississant d'association non ionique, amphiphile est choisi parmi les copolymères d'aminoplaste-polyéther modifiés de manière hydrophobe.

4. Agent selon la revendication 1, **caractérisé en ce que** l'épaississant d'association non ionique, amphiphile est choisi parmi les polymères de formule générale (I) 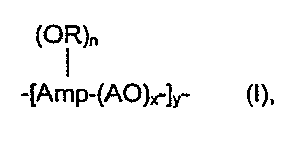 où Amp signifie un monomère d'aminoplaste ou le radical d'un oligomère ou d'un polymère d'aminoplaste, AO représente un groupe d'oxyde d'alkylène, R représente hydrogène, un groupe alkyle en C₁ à C₄ ou un groupe acyle en C₁ à C₄ et x et y sont des nombres supérieurs à 1.

5. Agent selon la revendication 1, **caractérisé en ce que** l'épaississant d'association non ionique, amphiphile est choisi parmi les produits de réaction de la polycondensation catalysée par un acide (a) de glycoluriles de formule générale (II), 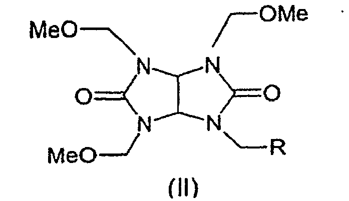 où R représente H ou de préférence OMe, avec (b) des poly(oxyde d'éthylène)diols présentant un degré d'éthoxylation de 20 à 500, ainsi que (c) d'un alcool, d'un alkylphénol, d'un thiol, d'un carboxamide, d'un carbamate hydrophobe, le cas échéant éthoxylé ou d'un acide carboxylique hydrophobe.

6. Agent selon la revendication 5, **caractérisé en ce que** le glycolurile de formule (II) est le 1,3,4,6-tétraméthoxy-méthylglycolurile.

7. Agent selon l'une quelconque des revendications 1 à 6, **caractérisé en ce que** l'épaississant d'association non ionique, amphiphile est choisi parmi le polyéther-1, le copolymère de PEG-180/octoxynol-40/TMMG et le copolymère de PEG-180/Laureth-50/TMMG.

8. Agent selon l'une quelconque des revendications 1 à 7, **caractérisé en ce qu'**il contient l'épaississant d'association non ionique amphiphile en une quantité de 0,01 à 2,5% en poids.

9. Agent selon l'une quelconque des revendications 1 à 8, **caractérisé en ce que** l'alcool gras est choisi parmi le décanol, l'undécanol, l'alcool laurylique, le 2-octyl-1-dodécanol, l'alcool oléylique, l'alcool linoléylique et l'alcool linolénylique.

10. Agent selon l'une quelconque des revendications 1 à 9, **caractérisé en ce qu'**il contient l'alcool gras en une quantité de 0,5 à 20% en poids.

11. Agent selon l'une quelconque des revendications 1 à 10, **caractérisé en ce qu'**il contient le copolymère de PEG-150/alcool stéarylique/SMDI en une quantité de 0,01 à 2% en poids.

12. Agent selon l'une quelconque des revendications 1 à 11, **caractérisé en ce qu'**il contient 0,01 à 12% en poids de précurseurs de colorant d'oxydation et est mélangé avant l'utilisation avec un oxydant.

13. Agent selon l'une quelconque des revendications 1 à 11, **caractérisé en ce qu'**il contient 0,01 à 12% en poids de précurseurs de colorant par oxydation, qui sont oxydables par l'oxygène de l'air.

14. Agent selon la revendication 1, **caractérisé en ce qu'**il contient (a) 0,01 à 2,5% en poids d'au moins un épaississant d'association non ionique, amphiphile, (b) 0,1 à 20% en poids d'au moins un alcool gras liquide à 25°C et (c) 0,01 à 2% en poids d'un copolymère de PEG-150/alcool stéarylique/SMDI, les composants (a) et (c) n'étant pas identiques, ainsi qu'au moins un précurseur d'un colorant par oxydation et un oxydant et est prêt à l'emploi.

15. Agent selon la revendication 14, **caractérisé en ce qu'**il s'agit d'un agent de teinture pour cheveux.
